# EUROPÄISCHE PATENTSCHRIFT

(11) **EP 3 201 528 B1**
(45) Veröffentlichungstag und Bekanntmachung des Hinweises auf die Patenterteilung: **24.04.2019**
(21) Anmeldenummer: 15793739.2
(22) Anmeldetag: 03.11.2015
(51) Int. Cl.: F21W 131/20, A61B 17/00, F21W 131/205

(54) **OPERATIONSLEUCHTE UND VERFAHREN ZUM BETREIBEN EINER OPERATIONSLEUCHTE**
SURGICAL LIGHT AND METHOD FOR THE USE OF A SURGICAL LIGHT
LAMPE SCYALITIQUE ET MÉTHODE D'OPÉRATION

(30) Priorität: 07.11.2014 DE 102014222794
(43) Veröffentlichungstag der Anmeldung: 09.08.2017
(73) Patentinhaber: TRUMPF Medizin Systeme GmbH + Co. KG, 07318 Saalfeld (DE)
(72) Erfinder: HARTL, Johannes, 85368 Moosburg (DE); GÜVENC, Deniz, 81737 München (DE); SCHMID,Michael, 88433 Schemmerhofen (DE); DOSER, Ingo, 79822 Titisee-Neustadt (DE)
(74) Vertreter: Prüfer & Partner mbB Patentanwälte · Rechtsanwälte
(86) Internationale Anmeldenummer: PCT/EP2015/075647
(87) Internationale Veröffentlichungsnummer: WO 2016/071371

(56) Entgegenhaltungen:
- EP-A1- 2 136 126
- EP-A1- 2 283 790
- CN-A- 101 858 537
- DE-T2- 60 300 647
- US-A1- 2003 185 009

## Beschreibung

Die Erfindung betrifft eine Operationsleuchte und ein Verfahren zum Betreiben einer Operationsleuchte, im Speziellen eine Operationsleuchte mit automatischen Einstellmöglichkeiten für ein Leuchtfeld und ein Verfahren zum Ausleuchten eines Operationsfelds an einem menschlichen Körper mit dieser Operationsleuchte.

Druckschrift US 6, 880, 957 B2 zeigt eine Operationsleuchte, bei der jede Lichtquelle mit einem Sensor ausgestattet ist. Der Sensor erkennt ein Hindernis nur im Strahlengang der jeweiligen Lichtquelle und dimmt diese Lichtquelle, um eine Schattenbildung des Hindernisses zu verhindern.

Dabei wird aber lediglich eine momentane Position des Hindernisses, nämlich nur eine Anwesenheit innerhalb des jeweiligen Strahlengangs, ausgewertet. Somit kann eine sich verändernde Verschattung des Operationsfelds nicht vorbestimmt werden und nicht im Voraus agiert werden.

Bisher werden außerdem für eine Abstandsmessung zwischen einem Leuchtenkörper der Operationsleuchte und dem Operationsfeld reine Abstandsmesseinrichtungen eingesetzt und für weitere sensorbasierte Anwendungen, wie beispielsweise die Einstellung von Fokuslagen und/oder Leuchtfeldabmessungen oder eine Gestensteuerung, werden spezielle zusätzliche Sensoren verwendet.

Dokument EP 2 283 790 A1 offenbart eine Steuerung und ein Verfahren zum Betreiben einer Operationsleuchte. Die Steuerung gibt ein Steuersignal zur Steuerung einer Richtung oder einer Intensität des Lichts oder für eine Größe oder Gestalt des von dem Licht beleuchteten Bereichs an die Operationsleuchte. Die Steuerung erkennt dazu durch ein Empfangen eines Bildsignals beispielsweise einen Handbereich von medizinischem Personal, um eine Position, eine Bewegung oder eine Geste zu erfassen, so dass diese in die Erzeugung des Steuersignals eingehen können. Die Steuerung kann ferner die Position von weiteren Körperteilen, wie dem Kopf des medizinischen Personals, erfassen, um beispielsweise mehrere Einzelleuchten so anzusteuern, dass diejenigen Einzelleuchten, deren Licht durch das medizinische Personal abgeschattet wird, abgeschaltet werden, und andere Einzelleuchten zugeschaltet werden.

In EP 2 136 126 A1 ist eine Operationsleuchte offenbart, die mehrere Leuchtmittel aufweist. Die Leuchtmittel erzeugen jeweils Lichtstrahlen, die entweder auf eine Mittelachse der Operationsleuchte gerichtet sind, oder alternativ nicht auf die Mittelachse gerichtet sind. Um ein rundes Leuchtfeld zu erzeugen, werden diejenigen Leuchtmittel betrieben, deren Lichtstrahlen sich auf der Mittelachse schneiden, wobei ein Abstand zwischen einem Leuchtenkörper und einer Operationsstelle über einen Abstandssensor gemessen wird, um einen Punkt mit maximalen resultierenden Helligkeit der Operationsleuchte durch Betrieben von jeweiligen Leuchtmitteln in dem Abstand von der Operationsstelle einzustellen.

DE 603 00 647 T2 zeigt eine Beleuchtungsvorrichtung für ein Operationsfeld. Die Beleuchtungsvorrichtung weist eine Vielzahl von Beleuchtungselementen und eine Einrichtung zum Erfassen eines Hindernisses auf. Beim Auftreten eines Hindernisses, das einen Schatten erzeugt, erhöht eine Zentraleinheit, mit Ausnahme der Beleuchtungselemente, in deren Lichtstrahl sich das Hindernis befindet, einen Sollwert der Lichtstärke der Beleuchtungselemente, die eine abgeschattete Überlappungszone aus Lichtstrahlen von mehreren Beleuchtungselementen beleuchten, so dass die gesamte Lichtstärke wieder ihrem Sollwert entspricht.

Im Dokument US 2003/0185009 A1 ist ebenfalls eine Beleuchtungsvorrichtung offenbart, die feststellt, wenn in Lichtstrahlen von einzelnen Beleuchtungselementen ein Hindernis vorhanden ist. Dazu weist die Beleuchtungsvorrichtung für jedes Beleuchtungselement einen Näherungsschalter auf und eine Steuerungsvorrichtung der Beleuchtungsvorrichtung steuert die einzelnen Beleuchtungselemente so an, dass die Beleuchtungselement, in deren Lichtstrahl das Hindernis ist, mit einer geringeren Leistung betrieben werden und diejenigen Beleuchtungselemente, die das Hindernis unterleuchten, mit einer größeren Leistung betrieben werden.

CN 101 858 537 A offenbart eine Operationsleuchte mit einem fünfdimensionalen Positioniermechanismus. Eine Position und Ausrichtung eines Leuchtenkörpers mit einer LED-Anordnung und eine Regulierung der LED-Anordnung wird in Echtzeit über einen Rechner gesteuert, um eine Schattenbildung zu vermeiden.

Die Erfindung beruht auf der Aufgabe, eine Operationsleuchte bereitzustellen, die die Voraussetzungen bietet, vorausschauend eine Schattenbildung zu verhindern oder zu reduzieren, und kostengünstig weitere Bedien- und Funktionsmöglichkeiten zu bieten.

Die Aufgabe wird durch eine Operationsleuchte gemäß Anspruch 1 und ein Verfahren gemäß Anspruch 11 gelöst. Weiterentwicklungen der Erfindungen sind Gegenstand der abhängigen Ansprüche.

Durch ein Vorsehen eines 3D-Sensors an einem Leuchtenkörper einer Operationsleuchte kann eine räumliche Position eines Gegenstands zwischen dem Leuchtenkörper und einem Operationsfeld sowie ein Abstand des Operationsfelds vom Leuchtenkörper als die Position erfasst werden.

Die Erfindung wird anhand von Ausführungsbeispielen unter Bezugnahme auf die beigefügten Zeichnungen erläutert.

Insbesondere zeigen
- Fig. 1: eine isometrische Ansicht eines Leuchtenkörpers einer Operationsleuchte; und
- Fig. 2: eine Seitenansicht des Leuchtenkörpers aus Fig. 1 mit exemplarischen Lichtstrahlen zum Bilden von Leuchtfeldern.

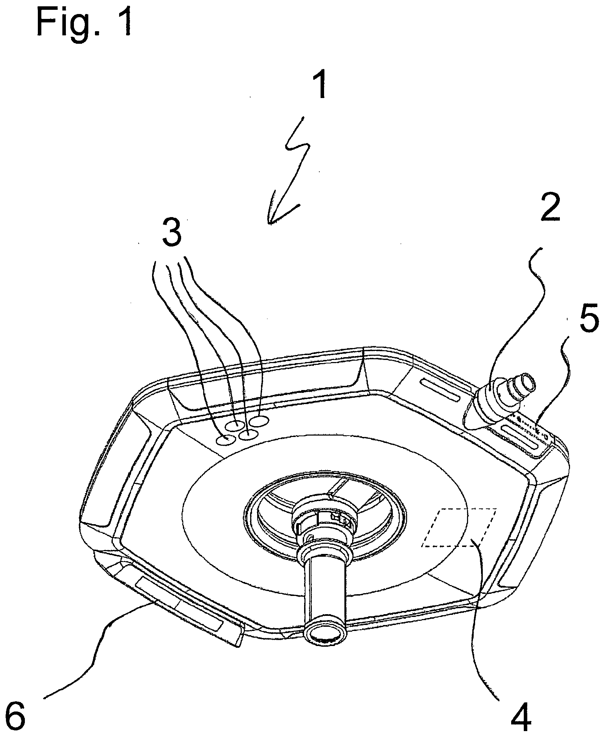

Fig. 1 zeigt einen Leuchtenkörper 1 einer Operationsleuchte. Der Leuchtenkörper 1 ist über ein Drehgelenk 2 mit einer nicht gezeigten Aufhängeeinrichtung beispielsweise an einer Raumdecke in allen Richtungen schwenkbar befestigt.

Der Leuchtenkörper 1 ist mit mehreren Leuchtmitteln 3 versehen. Die Leuchtmittel 3 befinden sich innerhalb des Leuchtenkörpers 1 und strahlen in der in Fig. 1 gezeigten Ausrichtung des Leuchtenkörpers 1 Licht in jeweils einem Lichtstrahl nach unten aus. Die Lichtstrahlen sind auf ein Operationsfeld an einem menschlichen Körper gerichtet, um dieses auszuleuchten. Von den Leuchtmitteln 3 sind hier nur vier von einer Mehrzahl dargestellt. Die Leuchtmittel 3 sind hier als LEDs ausgeführt und im Wesentlichen über eine gesamte Lichtaustrittsfläche am Leuchtenkörper 1 verteilt.

Die Operationsleuchte weist ferner im Leuchtenkörper 1, oder alternativ an einer anderen geeigneten Stelle, beispielsweise an einer Deckenbefestigung, eine Steuerungsvorrichtung 4 auf.

Die Operationsleuchte ist mit Bedienelementen 5, hier am Leuchtenkörper 1 versehen, wobei die Bedienelemente 5 auch an der Aufhängeeinrichtung oder einem nicht gezeigten Wandpanel vorgesehen sein können.

Die Leuchtmittel 3 sowie die Bedienelemente 5 sind mit der Steuerungsvorrichtung 4 verbunden. Die Steuerungsvorrichtung 4 steuert die Leuchtmittel 3 über ein nicht gezeigtes Mittel zum Ein- und Ausschalten und Dimmen der Leuchtmittel entsprechend Einstellungen an den Bedienelementen 5. Durch die Bedienelemente 5 ist ein Einstellen einer Intensität des von den Leuchtmitteln 3 ausgestrahlten Lichts, also einer Helligkeit in dem Operationsfeld, sowie eines Durchmessers eines durch die Leuchtmittel 3 in dem Operationsfeld erzeugten Leuchtfelds möglich. Optional sind weitere Einstellmöglichkeiten, beispielsweise eine Farbtemperatur des ausgestrahlten Lichts, möglich.

An dem Leuchtenkörper 1 ist ferner ein 3D-Sensor 6 zum Erfassen einer räumlichen Position von Gegenständen, wie Körperteilen von Operationspersonal, chirurgischen Geräten oder einem Körper eines Patienten, oder von Bewegungen der Gegenstände angeordnet. Der 3D-Sensor 6 ist mit der Steuerungsvorrichtung 4 verbunden und sendet entsprechend der erfassten Gegenstände Daten an die Steuerungsvorrichtung 4. Die Steuerungsvorrichtung 4 wertet die Daten des 3D-Sensors 6 aus und steuert die Leuchtmittel 3 entsprechend der Position und der Bewegung der Gegenstände an.

Alternativ kann der 3D-Sensor 6 die Position der Gegenstände bereits auswerten und sendet entsprechend aufbereitete Daten an die Steuerungsvorrichtung 4. In einer weiteren Alternative werden die Daten des 3-D-Sensors 6 durch diesen bis zu einem definierten Grad aufbereitet und dann durch die Steuerungsvorrichtung 4 endgültig verarbeitet. Dies ist dann vorteilhaft, wenn aus verschiedenen Quellen Daten, die beispielsweise gleiche Formateigenschaften haben, an die Steuerungsvorrichtung 4 geleitet werden. In einer weiteren alternativen Ausführungsform leitet der 3D-Sensor 6 Daten in unterschiedlichen Verarbeitungsstadien weiter, wenn diese, wie später beschrieben, für unterschiedliche Funktionalitäten unterschiedlich ausgewertet werden. Optional sind dann im Hinblick auf 3D-Sensordaten basierende Funktionalitäten der Operationsleuchte mehrere Steuerungsvorrichtungen 4 vorgesehen.

Optional erfasst der 3D-Sensor 6 zusätzlich eine Größe der erfassten Gegenstände. Aus den Daten bezüglich der Größe, der räumlichen Position und der Bewegung des Gegenstands steuert die Steuerungsvorrichtung 4 die Leuchtmittel 3 so, dass Lichtstrahlen, die auf einen erfassten Gegenstand zwischen dem Leuchtenkörper 1 und dem Operationsfeld treffen, gedimmt oder ausgeschaltet werden. Damit wird eine Schattenbildung durch den Gegenstand reduziert oder verhindert. Die Leuchtmittel 3, deren Lichtstrahlen nicht auf den Gegenstand treffen, sondern an dem Gegenstand vorbei auf das Leuchtfeld gerichtet sind, werden dann mit einer erhöhten Leistung betrieben, um eine Beleuchtungsstärke im Operationsfeld, also die Helligkeit, trotz des Gegenstands zwischen dem Leuchtenkörper 1 und dem Operationsfeld zumindest annähernd konstant zu halten. Je größer der Gegenstand ist, umso höher beispielsweise ist die Leistung, mit der die Leuchtmittel 3, deren Lichtstrahlen nicht auf den Gegenstand treffen, betrieben werden. Optional wird die Größe des Gegenstands auch mit dessen gemessenem Abstand in Verbindung gebracht. Bei einem geringen Abstand zu dem Leuchtenkörper 1 ist bei einer Fokussierung der Lichtstrahlen auf das Operationsfeld davon auszugehen, dass die Verschattung kleiner ist als bei Gegenständen in größeren Abständen zu dem Leuchtenkörper 1 bzw. zu dem 3D-Sensor 6.

Dazu ist in einem Speicherbereich der Steuerungsvorrichtung 4 für jedes der Leuchtmittel 3 ein Raum abgespeichert, der durch den Lichtstrahl ausgefüllt wird. Der Raum kann unter Berücksichtigung einer Abstandsinformation optional in weitere Abschnitte unterteilt werden. Wenn sich der Gegenstand zwischen dem Leuchtenkörper 1 und dem Operationsfeld zumindest teilweise in dem Raum des Lichtstrahls bzw. in einem der Abschnitte des Raums eines bestimmten Leuchtmittels 3 befindet, wird dieses Leuchtmittel 3 gedimmt oder ausgeschaltet. Das Ausmaß der Dimmung wird durch einen Anteil eines Querschnitts des Lichtstrahls bestimmt, der von dem Gegenstand eingenommen wird. Die Leuchtmittel 3, die an dieses bestimmte Leuchtmittel 3 angrenzen, werden dann mit einer höheren Leistung betrieben.

Optional wird auch eine Kontur des erfassten Gegenstands erfasst. Im Speicherbereich der Steuerungsvorrichtung 4 sind verschiedene Konturen den Gegenständen abgespeichert. Die Leuchtmittel 3 werden dann in Abhängigkeit von der Kontur des erfassten Gegenstands, also für eine Art des Gegenstands, unterschiedlich angesteuert. Dadurch kann beispielsweise für einen Typ von Operationsinstrument ein bestimmter Bereich des Operationsfelds mit einer größeren Beleuchtungsstärke beleuchtet werden. Bei einem Erkennen von beispielsweise einem Wundhaken wird der Randbereich des Operationsfelds stärker als andere Bereiche beleuchtet oder alternativ dort eine Schattenbildung verringert oder verhindert. Hingegen wird bei dem Erkennen eines Skalpells das Zentrum des Operationsfelds stärker als andere Bereiche beleuchtet oder alternativ dort eine Schattenbildung verringert oder verhindert. Es wird also durch die Steuerungsvorrichtung 4 auf Grund der Kontur vorausbestimmt, in welche Position sich der Gegenstand bewegen wird, und somit, auf welche Weise die Leuchtmittel 3 angesteuert werden.

Wiederum als eine Option bestimmt die Steuerungsvorrichtung 4 aus einer räumlichen Position des erfassten Gegenstands und seiner Bewegung, nämlich seiner Richtung und/oder seiner Geschwindigkeit und/oder seiner Beschleunigung eine Position, in die sich der Gegenstand bewegen wird, in der er sich also zu einem bestimmten nachfolgenden Zeitpunkt befinden wird. Dadurch ist es möglich, dass die Steuerungsvorrichtung 4 ohne einen Zeitverlust durch eine Reaktionszeit des 3D-Sensors, der Steuerungsvorrichtung 4 und der Leuchtmittel 3 eine Schattenbildung in Echtzeit vermeidet.

Ferner ist die Steuerungsvorrichtung 4 optional auch ausgebildet, über den 3D-Sensor einen Gegenstand zu erfassen, der sich außerhalb eines durch die Lichtstrahlen gebildeten Lichtkegels zwischen dem Leuchtenkörper 1 und dem Operationsfeld befindet, oder der sich darin in einer vorbestimmten Position befindet. Die Steuerungsvorrichtung 4 bestimmt dann aus einer Richtung und/oder einem Bahnverlauf und/oder einer Geschwindigkeit und/oder einer Beschleunigung einer Bewegung des erfassten Gegenstands in welcher Weise die Leuchtmittel 3 angesteuert werden. Damit ist es möglich, beispielsweise durch eine Gestensteuerung mit Hilfe einer Handbewegung, die Leuchtmittel 3 durch die Steuerungsvorrichtung 4 anzusteuern, um die Operationsleuchte zu dimmen oder den Leuchtfelddurchmesser zu verändern.

Der Leuchtfelddurchmesser ist so definiert, dass auf diesem Durchmesser eine Beleuchtungsstärke von 10% einer Beleuchtungsstärke im Zentrum des Leuchtfelds vorliegt.

Die Zuordnung von erfassten Gegenständen zu anzusteuernden Funktionen kann optional über eine Raumaufteilung und/oder spezifische Erkennungsmerkmale/Charakteristika erfolgen. Ferner kann über die erfassten Gegenstände entsprechend der Raumaufteilung auch ein Umschalten zwischen einem Vermeiden einer Schattenbildung und einer Gestensteuerung erfolgen. Bei der Raumaufteilung können verschiedene Bereiche in Richtung der Lichtstrahlen definiert werden. Beispielsweise werden im Nahfeld des Leuchtenkörpers, insbesondere in dem Bereich, in dem sich üblicherweise die Köpfe des Operationspersonals befinden, erkannte Gegenstände zu dem Vermeiden der Schattenbildung herangezogen. In einem mittleren Bereich, in dem sich üblicherweise Hände des Operationspersonals befinden, werden Gegenstände und deren Bewegung als Gesten für die Gestensteuerung verstanden. In einem Bereich auf oder direkt über dem Operationsfeld kann beispielsweise anhand dort befindlicher, im Voraus definierter Gegenstände bzw. Merkmale der Gegenstände, eine Ausrichtung der Lichtstrahlen erfolgen.

Alternativ kann auch ein von dem 3D-Sensor 6 erfasster aber nicht beleuchteter Bereich für die Gestensteuerung herangezogen werden, wohingegen innerhalb der Räume der Lichtstrahlen eine Schattenvermeidung und eine Ausrichtung der Lichtstrahlen, nämlich eine Position und ein Durchmesser des Leuchtfelds, durch eine räumliche Zuordnung der Gegenstände oder typische Charakteristika unterschieden werden. Beispielsweise werden für die Schattenvermeidung annähernd runde Gegenstände mit einer bestimmten Größe herangezogen, die auf einen Kopf schließen lassen, oder Gegenstände, die - beispielsweise in einer OP-Kopfbedeckung - eine Markierung aufweisen. Für eine Ausrichtung der Lichtstrahlen werden hingegen typische OP-Werkzeug- oder OP-Instrumentformen herangezogen.

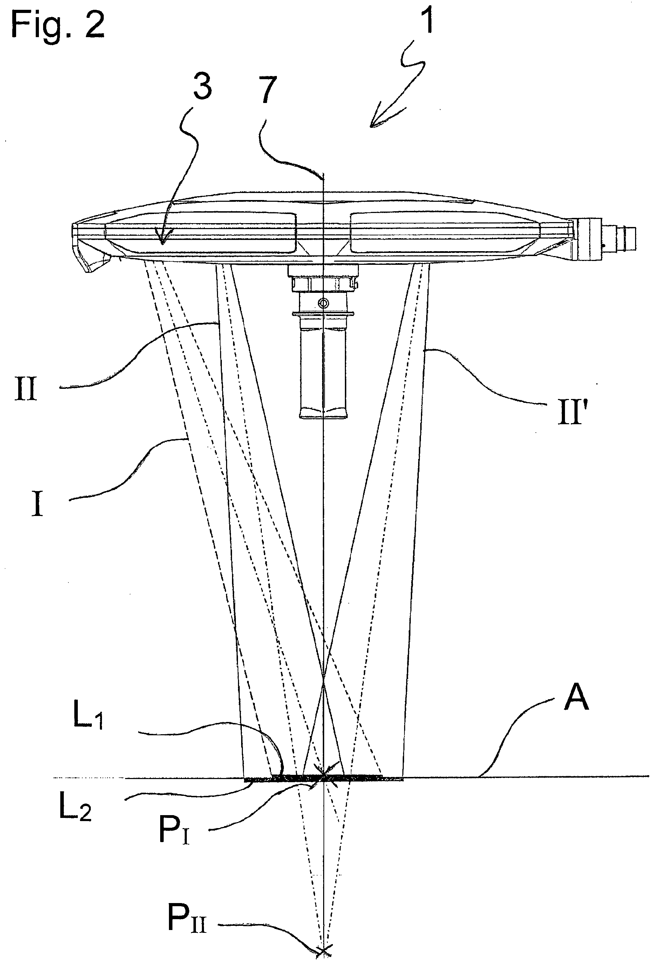

In Fig. 2 ist eine Seitenansicht des Leuchtenkörpers 1 gezeigt. Der Leuchtenkörper 1 hat eine Mittelachse 7. Die Leuchtmittel 3 in dem Leuchtenkörper 1 strahlen ihr Licht über Lichtstrahlen I, II, II' ab. Jedes der Leuchtmittel 3 in dem Leuchtenkörper 1 gibt einen der Lichtstrahlen I, II, II' ab, wobei in Fig. 2 für eine übersichtliche Darstellung lediglich die drei Lichtstrahlen I, II, II' dargestellt sind.

Um das Prinzip des Ausführungsbeispiels zu erklären, sind hier die Lichtstrahlen I, II, II' aller Leuchtmittel 3 auf die Mittelachse 7 des Leuchtenkörpers 1 gerichtet, wobei vereinfacht davon ausgegangen wird, dass Schnittpunkte P_{I}, P_{II} zwischen jedem Lichtstrahl I, II, II' und der Mittelachse gebildet werden. Die Lichtstrahlen schneiden, wie exemplarisch an den Lichtstrahlen I, II, II' gezeigt, die Mittelachse 7 in den Schnittpunkten P_{I}, P_{II} in unterschiedlichen Abständen. Auf Grund der vereinfachten Darstellung mit den drei Lichtstrahlen sind hier nur zwei Schnittpunkte P_{I}, P_{II} gezeigt, wobei dargestellt ist, dass auf den Schnittpunkt P_{I} lediglich der eine Lichtstrahl I gerichtet ist und auf den Schnittpunkt P_{II} zwei Lichtstrahlen II, II' gerichtet sind. Tatsächlich sind mehrere Schnittpunkte aus mehreren Lichtstrahlen mit der Mittelachse 7 vorhanden, wobei auch mehrere Lichtstrahlen (auch mehr als zwei Lichtstrahlen, wie an Hand von Schnittpunkt P_{II}, gezeigt), die Mittelachse 7 an demselben Schnittpunkt schneiden können. Tatsächlich können auch Lichtstrahlen vorgesehen sein, die nicht auf die Mittelachse 7 gerichtet sind.

Der Lichtstrahl I bildet auf der Operationsstelle ein Leuchtfeld L₁ und die Lichtstrahlen II, II' bilden auf der Operationsstelle ein Leuchtfeld L₂. Die Lichtstrahlen I, II, II' sind überlagert, um ein daraus resultierendes Leuchtfeld zu bilden, das einen bestimmten Leuchtfelddurchmesser und eine bestimmte Lichtverteilung im Leuchtfeld hat. Durch eine unterschiedliche Ansteuerung der Leuchtmittel 3 durch die Steuerungsvorrichtung 4 ist es möglich, den Leuchtfelddurchmesser und die Lichtverteilung im resultierenden Leuchtfeld einzustellen.

Durch die waagerechte Linie A in Fig. 2 ist vereinfacht eine Oberfläche des menschlichen Körpers dargestellt, auf dem sich das Operationsfeld befindet. Der menschliche Körper, der in diesem Zusammenhang als der erfasste Gegenstand angesehen wird, ist in einer räumlichen Position, die als Abstand zwischen dem Leuchtenkörper 1 und dem Operationsfeld auf der Oberfläche A des menschlichen Körpers erfasst wird.

Der Lichtstrahl I ist auf den Schnittpunkt P_{I} gerichtet, der sich im Abstand des Operationsfelds von dem Leuchtenkörper 1 auf der Mittelachse 7 befindet. Die Lichtstrahlen II, II' sind auf den Schnittpunkt P_{II}, gerichtet, der sich nicht im Abstand des Operationsfelds von dem Leuchtenkörper 1 auf der Mittelachse befindet.

Um die Lichtstrahlen I, II, II' auf dem Operationsfeld zu fokussieren, wird von der Steuerungsvorrichtung 4 ausschließlich, oder mit einer gegenüber den anderen Leuchtmitteln 3 erhöhter Leistung, dasjenige Leuchtmittel 3 betrieben, dessen Lichtstrahlen I auf den Schnittpunkt P_{I} gerichtet sind, dessen Abstand gleich dem Abstand des Operationsfelds von dem Leuchtenkörper 1 ist, und der das Lichtfeld L₁ bildet. Falls kein Schnittpunkt P_{I}, P_{II} im Abstand von dem Operationsfeld existiert, werden diejenigen Leuchtmittel 3 betrieben, deren Lichtstrahlen I, II, II' einen Schnittpunkt P₁, P₂ bilden, dessen Abstand von dem Leuchtenkörper 1 dem Abstand des Operationsfelds von dem Leuchtenkörper 1 am nächsten ist. Entweder werden diese Leuchtmittel 3 ausschließlich oder gemeinsam mit anderen Leuchtmitteln 3, jedoch dann mit einer gegenüber den anderen Leuchtmitteln 3 erhöhten Leistung, betrieben.

Um andererseits einen Leuchtfelddurchmesser mittels unterschiedlicher Ansteuerungen der Leuchtmittel 3 einstellen zu können, steuert die Steuerungsvorrichtung 4 die Leuchtmittel 3 so an, dass dasjenige Leuchtmittel 3, dessen Lichtstrahl I die Mittelachse 7 im Abstand des Operationsfelds schneidet, als auch diejenigen Leuchtmittel 3, deren Lichtstrahl II, II' die Mittelachse 7 nicht im Abstand des Operationsfelds schneidet, mit aufeinander abgestimmten Leistungen betrieben werden. Für einen möglichst kleinen Leuchtfelddurchmesser werden, wie oben, beschrieben, diejenigen Leuchtmittel 3 betrieben, deren Lichtstrahl I die Mittelachse im Abstand des Operationsfelds schneiden, um das Lichtfeld L₁ zu bilden. Für eine Vergrößerung des Durchmessers des Leuchtfelds L₁, L₂ werden dann diejenigen Leuchtmittel 3 mit erhöhter Leistung betrieben, deren Lichtstrahlen II, II' die Mittelachse 7 nicht im Abstand des Operationsfeld schneidet. Durch diese Lichtstrahlen II, II' wird das Lichtfeld L₂, dessen Durchmesser größer als der des Lichtfelds L₁ ist, gebildet. Um einen Leuchtfelddurchmesser zu bilden, der größer als der des Leuchtfelds L1 aber der kleiner als der Durchmesser des Leuchtfelds L₂ ist, werden sowohl die Leuchtmittel 3, die das Lichtfeld L₁ bilden, als auch die Leuchtmittel 3, die das Lichtfeld L₂ bilden, mit abgestimmter Leistung betrieben. Dabei kann bei einem gleichzeitigen Betrieb der Leuchtmittel 3, die das Lichtfeld L₁ bilden, als auch der Leuchtmittel 3, die das Lichtfeld L₂ bilden, ein daraus resultierendes Leuchtfeld gebildet werden, dessen resultierender Leuchtfelddurchmesser zwischen den Durchmessern des Leuchtfelds L₁ und des Leuchtfelds L₂ liegt. Je höher der Anteil der ausgestrahlten Intensität der Leuchtmittel 3, die die bestimmten Leuchtfelddurchmesser L₁ und L₂ bilden, an einer Gesamtleistung ist, umso näher liegt der resultierende Leuchtfelddurchmesser bei diesem bestimmten Leuchtfelddurchmesser. Durch eine abgestimmte Leistung, mit der die Leuchtmittel 3 betrieben werden, ist daher einerseits der resultierende Leuchtfelddurchmesser einstellbar, andererseits auch die Beleuchtungsstärke, also die Helligkeit, im Operationsfeld einstellbar.

Optional ist es auch möglich, durch die Messung des Abstands des Leuchtenkörpers 1 von der Operationsstelle, die Leuchtmittel 3 so anzusteuern, dass die Beleuchtungsstärke im Zentrum des Operationsfelds bei einer Veränderung des Abstands konstant bleibt. Die zugehörigen Leistungswerte werden dabei entweder empirisch ermittelt und in dem Speicherbereich der Steuerungsvorrichtung 4 abgespeichert, oder jeweils über einen Algorithmus errechnet.

In einer weiteren Option wird durch den 3D-Sensor 6 eine Topographie des Operationsfelds erfasst. Aus den Eigenschaften des Operationsfelds, wie etwa der Größe, der Tiefe oder der Neigung, bestimmt die Steuerungsvorrichtung 4, welche Leuchtmittel 3 betrieben werden, um das Operationsfeld möglichst optimal auszuleuchten. Bei großflächigen Operationsfeldern werden beispielsweise diejenigen Leuchtmittel 3 betrieben, die ein Leuchtfeld L₁, L₂ mit großem Durchmesser erzeugen. Im Fall von kleinen Operationsfeldern werden diejenigen Leuchtmittel 3 betrieben, die ein Leuchtfeld L₁, L₂ mit kleinem Durchmesser erzeugen. Der Durchmesser des Leuchtfelds L₁, L₂ entspricht dabei im Wesentlichen der Größe des Operationsfelds. Im Falle von tiefen Operationsfeldern werden diejenigen Leuchtmittel 3 betrieben, deren Lichtstrahl I, II, II' möglichst senkrecht auf das Operationsfeld gerichtet ist, um die gesamte Tiefe des Operationsfelds auszuleuchten.

In einer weiteren alternativen Ausführungsform wird die Operationsleuchte in einem System von mindestens zwei Operationsleuchten verwendet.

In einem Fall, in dem die Lichtstrahlen auf das gleiche Operationsfeld gerichtet sind, besteht die Gefahr, dass, obwohl jede der Operationsleuchten für sich die zulässigen Bereiche für die Beleuchtungsstärke im Operationsfeld einhält, durch eine Überlagerung der Leuchtfelder von mehreren Operationsleuchten die maximal zulässige Beleuchtungsstärke überschritten wird. Dadurch besteht die Gefahr einer Blendung des Operationspersonals sowie einer Austrocknung der Wunde im Operationsfeld.

Um dies zu vermeiden, wird optional mit den 3D-Sensoren 6 der einzelnen Operationsleuchten jeweils die räumliche Anordnung des Operationsfelds erkannt. Über die jeweiligen Steuerungsvorrichtungen 4 der Operationsleuchten wird bestimmt, ob die Lichtstrahlen I, II, II' der Leuchtmittel 3 der jeweiligen Operationsleuchte auf das gleiche Operationsfeld gerichtet sind und darauf ein Leuchtfeld L₁, L₂ bilden. Sofern von den Steuerungsvorrichtungen 4 bestimmt wird, dass die Lichtstrahlen I, II, II' auf dasselbe Operationsfeld gerichtet sind, wird durch die Steuerungsvorrichtungen 4 der Operationsleuchten die Leistung der auf das Operationsfeld gerichteten Leuchtmittel 3 so abgestimmt, dass die maximal zulässige Beleuchtungsstärke, oder optional eine einstellbare maximale Beleuchtungsstärke, nicht überschritten wird.

Optional besteht auch die Möglichkeit, die tatsächliche Beleuchtungsstärke in dem Operationsfeld mittels eines andersartigen Sensors, beispielsweise eines Helligkeitssensors, zu erfassen, oder eine Temperatur in dem Operationsfeld über einen Temperatursensor zu erfassen, um das Austrocknen der Wunde zu vermeiden.

Weiterhin besteht in dem Fall, in dem mehrere Operationsleuchten auf dasselbe Operationsfeld gerichtet sind, die Möglichkeit, die Schattenvermeidung nicht nur durch die Ansteuerung der Leuchtmittel 3 von dem Leuchtenkörper 1, dessen 3D-Sensor den Gegenstand erfasst, zu erzielen. Bei dem Erfassen des Gegenstands zwischen dem Leuchtenkörper 1 und dem Operationsfeld werden einerseits zusätzlich zu dem Dimmen oder Abschalten der Leuchtmittel 3, in deren Lichtstrahl I, II, II' der Gegenstand liegt, die anderen Leuchtmittel 3 desselben Leuchtenkörpers 1 durch die Steuerungsvorrichtung 4 mit einer erhöhten Leistung betrieben. Weiterhin steuern die Steuerungsvorrichtungen 4 der anderen Operationsleuchten, die ebenfalls auf dasselbe Leuchtfeld L₁, L₂ gerichtet sind, abgestimmt mit der Steuerungsvorrichtung 4 der Operationsleuchte, deren 3D-Sensor den Gegenstand erfasst, ihre Leuchtmittel 3 mit einer erhöhten Leistung an, sofern kein Gegenstand in deren jeweiligen Lichtstrahlen I, II, II' liegt.

Optional steuern die Steuerungsvorrichtungen 4 der einzelnen Operationsleuchten aufeinander abgestimmt die Leuchtmittel 3 so an, dass die Beleuchtungsstärke in dem Operationsfeld zumindest annähernd konstant bleibt.

Im Betrieb wird eine räumliche Position von mindestens einem der Gegenstände, von einem der Körperteile vom Operationspersonal oder von einem der chirurgischen Geräte, von dem 3D-Sensor 6 erfasst. Die entsprechenden Daten bezüglich der räumlichen Position des Gegenstands werden an die Steuerungsvorrichtung 4 gesendet. Anschließend werden die Leuchtmittel 3 entsprechend der von dem 3D-Sensor erfassten räumlichen Position angesteuert.

Optional wird auch die Größe des sich zwischen dem Leuchtenkörper 1 und dem Operationsfeld befindlichen Gegenstands erfasst. Diejenigen Leuchtmittel 3, deren Lichtstrahl I, II, II' auf den Gegenstand gerichtet ist, werden dann gedimmt oder ausgeschaltet, um eine Schattenbildung durch den Gegenstand zu verhindern. Um die durch das Ausschalten oder das Dimmen entstehende verminderte Helligkeit im Operationsfeld auszugleichen, werden diejenigen Leuchtmittel 3, deren Lichtstrahlen I, II, II' an dem Gegenstand vorbei auf das Operationsfeld gerichtet sind, mit einer erhöhten Leistung betrieben, so dass die Helligkeit im Operationsfeld zumindest annähernd konstant bleibt.

Wenn, zusätzlich zu der räumlichen Position, eine Bewegung oder eine Kontur des Gegenstands erfasst wird, kann vorausbestimmt werden, welche Position der Gegenstand zu einem bestimmten Zeitpunkt einnehmen wird. So kann beispielsweise aus einer Geschwindigkeit oder einer Beschleunigung in einer bestimmten Richtung diese vorausbestimmte Position zu einem bestimmten Zeitpunkt berechnet werden. Die Leuchtmittel 3 werden dann durch die Steuerungsvorrichtung 4 so angesteuert, dass die Leuchtmittel 3, deren jeweiliger Lichtstrahl zu diesem Zeitpunkt auf die vorausbestimmte Position gerichtet ist, nicht oder nur gedimmt betrieben wird. Die Lichtstrahlen, die dann nicht auf die vorbestimmte Position des Gegenstands gerichtet sind und dann an dem Gegenstand vorbei auf das Operationsfeld gerichtet sind, werden, wie oben erläutert, mit erhöhter Leistung betrieben.

Die Bewegung des erfassten Gegenstands wird optional nicht nur zur Schattenvermeidung verwendet, sondern auch, um die Operationsleuchte zu steuern. So wird beispielsweise eine durch den gleichen 3D-Sensor 6 erfasste Bewegung einer Hand, also einer Geste eines Operateurs verwendet, um entsprechend ihrer Richtung, ihres Bahnverlaufs, ihrer Geschwindigkeit oder ihrer Beschleunigung, eine bestimmte Ansteuerung der Leuchtmittel 3, beispielsweise ein Ausschalten oder Dimmen, durch die Steuerungsvorrichtung 4 auszuführen.

Um die auf das Operationsfeld gerichteten Lichtstrahlen zu fokussieren, werden durch die Steuerungsvorrichtung 4 nur oder hauptsächlich diejenigen Leuchtmittel 3 betrieben, die auf den Schnittpunkt gerichtet sind, in dem deren Lichtstrahl I die Mittelachse 7 im Abstand des Operationsfelds schneidet, oder auf einen Punkt, der dem Abstand des Operationsfelds am nächsten ist.

Um den Leuchtfelddurchmesser auf einen gewünschte Größe einzustellen, werden die auf einen Punkt auf der Mittelachse 7 im Abstand des Operationsfelds gerichteten Leuchtmittel 3 und auch die nicht auf den Punkt auf der Mittelachse 7 im Abstand des Operationsfelds gerichteten Leuchtmittel 3, wie oben beschrieben, mit der abgestimmten Leistung betrieben.

Um eine Bedienung der Operationsleuchte zu vereinfachen, werden die Leuchtmittel 3 von der Steuerungsvorrichtung 4 so angesteuert, dass bei einer Veränderung des Abstands zwischen dem Leuchtenkörper 1 und dem Operationsfeld die Beleuchtungsstärke im Zentrum des Leuchtfelds L₁, L₂ konstant bleibt.

Um das Operationsfeld möglichst optimal auszuleuchten, wird die Topographie des Operationsfelds durch den 3D-Sensor 6 erfasst. Aus den Eigenschaften des Operationsfelds, wie etwa der Größe, der Tiefe oder der Neigung, bestimmt die Steuerungsvorrichtung 4, welche Leuchtmittel 3 betrieben werden, um das Operationsfeld wie oben beschrieben auszuleuchten.

Die unterschiedlichen Ausführungsformen sind miteinander kombinierbar.

## Patentansprüche

1. Operationsleuchte zum Ausleuchten eines Operationsfelds auf einem menschlichen Körper mit
einer Steuerungsvorrichtung (4),
einem Leuchtenkörper (1), der
mehrere Leuchtmittel (3) mit jeweils einem auf das Operationsfeld gerichteten Lichtstrahl (I, II, II'), und
einen 3D-Sensor (6) zum Erfassen einer räumlichen Position von mindestens einem Gegenstand aufweist, und
Mittel zum Ein- und Ausschalten und Dimmen der Leuchtmittel (3),
wobei die Steuerungsvorrichtung (4) die Mittel zum Ein- und Ausschalten und Dimmen der Leuchtmittel (3) ansteuert,
der 3D-Sensor (6) die räumliche Position und eine Größe des mindestens einen Gegenstands zwischen dem Leuchtenkörper (1) und dem Operationsfeld erfasst und entsprechende Daten an die Steuerungsvorrichtung (4) sendet,
die Steuerungsvorrichtung (4) angepasst ist,
die Leuchtmittel (3) entsprechend der räumlichen Position des mindestens einen Gegenstands anzusteuern, und
auf Grund der räumlichen Position und der Größe des mindestens einen Gegenstands diejenigen Leuchtmittel (3) zu dimmen oder auszuschalten, deren jeweiliger Lichtstrahl (I, II, II') auf den mindestens einen Gegenstand gerichtet ist, und diejenigen Leuchtmittel (3) mit erhöhter Leistung zu betreiben, deren jeweiliger Lichtstrahl (I, II, II') an dem mindestens einen Gegenstand vorbei auf das Operationsfeld gerichtet ist, wobei
der 3D-Sensor (6) angepasst ist, eine Bewegung des mindestens einen Gegenstands zu erfassen, und die Steuerungsvorrichtung (4) angepasst ist, die Leuchtmittel (3) entsprechend der Bewegung des mindestens einen Gegenstands anzusteuern, und **dadurch gekennzeichnet dass**, die Steuerungsvorrichtung (4) angepasst ist, aus der räumlichen Position und der Bewegung, und/oder einer Kontur des mindestens einen Gegenstands eine voraussichtliche Position des mindestens einen Gegenstands zu einem bestimmten Zeitpunkt vorauszubestimmen, und die Leuchtmittel (3) in Abhängigkeit von der vorausbestimmten Position und der Größe des mindestens einen Gegenstands so zu steuern, dass zu dem bestimmten Zeitpunkt diejenigen Leuchtmittel (3) gedimmt oder ausgeschaltet werden, deren jeweiliger Lichtstrahl (I, II, II') dann auf den mindestens einen Gegenstand in der vorausbestimmten Position gerichtet ist, und diejenigen Leuchtmittel mit erhöhter Leistung zu betreiben, deren jeweiliger Lichtstrahl (I, II, II') dann an dem mindestens einen Gegenstand in der vorausbestimmten Position vorbei auf das Operationsfeld gerichtet ist.

2. Operationsleuchte gemäß Anspruch 1, wobei die Steuerungsvorrichtung (4) angepasst ist, aus einer Kontur des mindestens einen Gegenstands einen Bereich des Operationsfelds zu bestimmen, der stärker zu beleuchten ist, und die Steuerungsvorrichtung (4) angepasst ist, diejenigen Leuchtmittel (3) mit einer erhöhten Leistung zu betreiben, deren jeweiliger Lichtstrahl (I, II, II') auf den Bereich gerichtet ist.

3. Operationsleuchte gemäß Anspruch 1 oder 2, wobei die Steuerungsvorrichtung (4) angepasst ist, aus einer Richtung der Bewegung des mindestens einen Gegenstands die vorausbestimmte Position vorauszubestimmen.

4. Operationsleuchte gemäß eine der Ansprüche 1 bis 3, wobei die Steuerungsvorrichtung angepasst ist, aus einer Geschwindigkeit der Bewegung des mindestens einen Gegenstands die vorausbestimmte Position vorauszubestimmen.

5. Operationsleuchte gemäß einem der Ansprüche 1 bis 3, wobei die Steuerungsvorrichtung angepasst ist, aus einer Beschleunigung der Bewegung des mindestens einen Gegenstands die vorausbestimmte Position vorauszubestimmen.

6. Operationsleuchte gemäß einem der Ansprüche 1 bis 5, wobei die Steuerungsvorrichtung (4) angepasst ist, die Leuchtmittel (3) entsprechend einer Richtung, einem Bahnverlauf, einer Geschwindigkeit oder einer Beschleunigung der Bewegung des mindestens einen Gegenstands zu steuern.

7. Operationsleuchte gemäß einem der vorangehenden Ansprüche,
wobei einer der Gegenstände der menschliche Körper ist, auf dem sich das Operationsfeld befindet,
die räumliche Position dem Abstand des Operationsfelds von dem Leuchtenkörper (1) entspricht,
der Leuchtenkörper (1) eine Mittelachse (7) aufweist,
die Lichtstrahlen (I, II, II') auf Schnittpunkte (P_{I}, P_{II}) mit der Mittelachse (7) gerichtet sind, die unterschiedliche Abstände vom Leuchtenkörper (1) haben,
die Lichtstrahlen (I, II, II') überlagert sind, um ein Leuchtfeld (L₁, L₂) auf dem Operationsfeld bilden, und
die Steuerungsvorrichtung (4) angepasst ist, die Leuchtmittel (3) so anzusteuern, dass diejenigen Leuchtmittel (3), deren jeweiliger Lichtstrahl (I, II, II') die Mittelachse (7) im Abstand des Operationsfelds schneidet, oder der auf einen Punkt gerichtet ist, dessen Abstand dem Abstand des Operationsfelds am nächsten ist, mit einer gegenüber den verbleibenden Leuchtmitteln (3) erhöhten Leistung betrieben werden, um die Lichtstrahlen (I, II, II') auf der Operationsstelle zu fokussieren.

8. Operationsleuchte gemäß Anspruch 7, wobei
die Steuerungsvorrichtung (4) angepasst ist, die Leuchtmittel (3) so anzusteuern, dass alternativ sowohl diejenigen Leuchtmittel (3), deren Lichtstrahl (I, II, II') die Mittelachse (7) im Abstand des Operationsfelds schneidet, als auch diejenigen, deren Lichtstrahl (I, II, II') die Mittelachse (7) nicht im Abstand des Operationsfelds schneidet, mit einer aufeinander abgestimmten Leistung betrieben werden, um einen Leuchtfelddurchmesser einstellbar zu machen.

9. Operationsleuchte gemäß Anspruch 7 oder 8, wobei die Steuerungsvorrichtung (4) angepasst ist, die Leuchtmittel (3) so anzusteuern, dass eine Beleuchtungsstärke im Zentrum des Operationsfelds (L₁, L₂) bei einer Veränderung des Abstands des Operationsfelds konstant bleibt.

10. Operationsleuchte gemäß einem der vorangehenden Ansprüche, wobei der 3D-Sensor (6) angepasst ist, eine Oberflächenstruktur des Operationsfelds zu erfassen, und die Steuerungsvorrichtung (4) angepasst ist, aus den durch den 3D-Sensor gesendeten Daten eine Topographie des Operationsfelds zu bestimmen und die Leuchtmittel (3) entsprechend der Topographie zu betreiben.

11. Verfahren zum Betreiben einer Operationsleuchte gemäß einem der Ansprüche 1 bis 10, das folgende Schritte aufweist
- Erfassen der räumlichen Position des mindestens einen Gegenstands durch den 3D-Sensor (6) und Senden der entsprechenden Daten an die Steuerungsvorrichtung (4);
- Ansteuern der Leuchtmittel (3) entsprechend der räumlichen Position des mindestens einen Gegenstands;
- Erfassen der Größe des mindestens einen Gegenstands zwischen dem Leuchtenkörper (1) und dem Operationsfeld mittels des 3D-Sensors (6);
- Dimmen oder Ausschalten derjenigen Leuchtmittel (3), deren jeweiliger Lichtstrahl auf den mindestens einen Gegenstand gerichtet ist durch die Steuerungsvorrichtung (4); und
- Betreiben der Leuchtmittel mit erhöhter Leistung, deren Lichtstrahlen an dem mindestens einen Gegenstand vorbei auf das Operationsfeld gerichtet sind, durch die Steuerungsvorrichtung (4) ; und **gekennzeichnet durch**
- Vorausbestimmen einer Position des mindestens einen Gegenstands zu dem bestimmten Zeitpunkt aus der räumlichen Position und der Bewegung und/oder einer Kontur des mindestens einen Gegenstands mittels der Steuerungsvorrichtung (4) ; und
- Ansteuern der Leuchtmittel (3) in Abhängigkeit von der vorausbestimmten Position und der Größe des mindestens einen Gegenstands, so dass zu dem bestimmten Zeitpunkt diejenigen Leuchtmittel (3) gedimmt oder ausgeschaltet werden, deren jeweiliger Lichtstrahl (I, II, II') auf den mindestens einen Gegenstand in der vorbestimmten Position gerichtet ist, und diejenigen Leuchtmittel (3) mit erhöhter Leistung betrieben werden, deren jeweiliger Lichtstrahl (I, II, II') an dem mindestens einen Gegenstand in der vorbestimmten Position vorbei auf das Operationsfeld gerichtet ist.

12. Verfahren gemäß Anspruch 11 mit einer Operationsleuchte gemäß Anspruch 3 oder 4, wobei, die vorausbestimmte Position aus der Richtung, der Geschwindigkeit oder der Beschleunigung des mindestens einen Gegenstands durch die Steuerungsvorrichtung (4) vorausbestimmt wird.

13. Verfahren gemäß einem der Ansprüche 11 oder 12, mit einer Operationsleuchte gemäß Anspruch 5 mit dem Schritt
- Ansteuern der Leuchtmittel (3) entsprechend einer Richtung, einem Bahnverlauf, einer Geschwindigkeit oder einer Beschleunigung der Bewegung des mindestens einen Gegenstands durch die Steuerungsvorrichtung (4).

14. Verfahren gemäß einem der Ansprüche 11 bis 13 mit einer Operationsleuchte gemäß einem der Ansprüche 7 bis 10, mit dem Schritt
- Ansteuern der Leuchtmittel (3) so, dass diejenigen Leuchtmittel (3), deren Lichtstrahl. (I, II, II') die Mittelachse (7) im Abstand des Operationsfelds schneidet, oder auf den Punkt gerichtet ist, dessen Abstand dem Abstand des Operationsfelds am nächsten ist, mit einer gegenüber den verbleibenden Leuchtmitteln (3) erhöhten Leistung betrieben werden, um die Lichtstrahlen (I, II, II') auf der Operationsstelle zu fokussieren.

15. Verfahren gemäß einem der Ansprüche 11 bis 14 mit einer Operationsleuchte gemäß einem der Ansprüche 6 bis 9, mit dem Schritt
- Ansteuern der Leuchtmittel (3) so, dass sowohl diejenigen Leuchtmittel (3), deren Lichtstrahl die Mittelachse (7) im Abstand des Operationsfelds schneidet, als auch diejenigen Leuchtmittel (3), deren Lichtstrahl (I, II, II') die Mittelachse (7) nicht im Abstand des Operationsfelds schneidet, mit einer aufeinander abgestimmten Leistung durch die Steuerungsvorrichtung (4) betrieben werden, um einen Leuchtfelddurchmesser einstellbar zu machen.

16. Verfahren gemäß einem der Ansprüche 11 bis 15 mit einer Operationsleuchte gemäß einem der Ansprüche 6 bis 9, mit dem Schritt
- Ansteuern der Leuchtmittel (3) so, dass eine Beleuchtungsstärke im Zentrum des Operationsfelds bei einer Veränderung des Abstands des Operationsfelds konstant bleibt.

17. Verfahren gemäß einem der Ansprüche 11 bis 16 mit einer Operationsleuchte gemäß Anspruch 9, mit den Schritten
- Erfassen einer Topographie durch die Steuerungsvorrichtung (4) aus den von dem 3D-Sensor (6) gesendeten Daten;
- Betreiben der Leuchtmittel (3) durch die Steuerungsvorrichtung (4) so, dass das Operationsfeld entsprechend der Topographie ausgeleuchtet wird.

18. Verfahren gemäß Anspruch 17, wobei das Operationsfeld durch die Leuchtmittel (3) ausgeleuchtet wird, die ein Leuchtfeld (L₁, L₂) mit einem großen Durchmesser bilden, wenn das Operationsfeld großflächig ist, und das Operationsfeld durch die Leuchtmittel (3) ausgeleuchtet werden, die ein Leuchtfeld (L₁, L₂) mit einem kleinen Durchmesser bilden, wenn das Operationsfeld klein ist, so dass der Durchmesser des Leuchtfelds (L₁, L₂) im Wesentlichen der Größe des Operationsfelds entspricht.

19. Verfahren gemäß Anspruch 17 oder 18, wobei das Operationsfeld durch Leuchtmittel (3) ausgeleuchtet wird, deren Lichtstrahlen (I, II, II') möglichst senkrecht auf das Operationsfeld gerichtet sind, wenn das Operationsfeld eine tiefe Wunde enthält.

20. System aus mehreren Operationsleuchten mit zumindest einer Operationsleuchte gemäß einem der Ansprüche 1 bis 10, wobei die Steuerungsvorrichtungen (4) mehrerer Operationsleuchten dazu angepasst sind, über die Daten des 3D-Sensors (6) angesteuert zu werden.

## Claims

1. Surgical lamp for illuminating a surgical field on a human body having
a control device (4),
a lamp body (1) comprising
several illuminants (3) with one respective light beam (I, II, II') directed to the surgical field, and
a 3D sensor (6) for detecting a spatial position of at least one object, and
devices for switching on and off and dimming the illuminants (3),
wherein the control device (4) controls the devices for switching on and off and dimming the illuminants (3),
the 3D sensor (6) detects the spatial position and a size of the at least one object between the lamp body (1) and the surgical field and transmits corresponding data to the control device (4),
the control device (4) is adapted
to control the illuminants (3) according to the spatial position of the at least one object, and
due to the spatial position and the size of the at least one object, to dim or to switch off the ones of the illuminants (3), the respective light beam (I, II, II') of which is directed to the at least one object, and to operate the ones of the illuminants (3), the respective light beam (I; II; II') of which is directed past the at least one object to the surgical field, with increased performance, wherein
the 3D sensor (6) is adapted to detect a motion of the at least one object, and the control device (4) is adapted to control the illuminants (3) according to the motion of the at least one object, and
**characterized in that**
the control device (4) is adapted to predetermine an expected position of the at least one object at a certain point of time from the spatial position and the motion and/or a contour of the at least one object and to control the illuminants (3) depending on the predetermined positon and the size of the at least one object such that the ones of the illuminants (3), the respective light beam (I, II; II') of which is then directed to the at least one object in the predetermined position, are dimmed or switched off at the certain point of time, and the ones of the illuminants, the respective light beam (I; II, II') of which is then directed past the at least one object in the predetermined position to the surgical field, are operated with an increased performance.

2. Surgical lamp according to claim 1, wherein the control device (4) is adapted to determine an area of the surgical field which is to be illuminated in an increased manner from a contour of the at least one object, and the control device (4) is adapted to operate the ones of the illuminants (3), the respective light beam (I, II, II') of which are directed to the area, with an increased performance.

3. Surgical lamp according to claim 1 or 2, wherein the control device (4) is adapted to predetermine the predetermined position from a direction of the motion of the at least one object.

4. Surgical lamp according to anyone of claims 1 to 3, wherein the control device is adapted to predetermine the predetermined position from a velocity of the motion of the at least one object.

5. Surgical lamp according to anyone of claim 1 to 3, wherein the control device is adapted to predetermine the predetermined position from an acceleration of the motion of the at least one object.

6. Surgical lamp according to anyone of the claims 1 to 5, wherein the control device (4) is adapted to control the illuminants (3) according to a direction, a trajectory, a velocity, or an acceleration of the motion of the at least one object.

7. Surgical lamp according to anyone of the preceding claims,
wherein one of the objects is the human body on which the surgical field is located,
the spatial position corresponds to the distance of the surgical field from the lamp body (1),
the lamp body (1) comprises a central axis (7),
the light beams (I, II, II') are directed to intersection points (P_{I}, P_{II}) with the central axis (7), the intersection points having different distances from the lamp body (1),
the light beams (I, II, II') are superimposed in order to generate a light field (L₁, L₂) on the surgical field, and
the control device (4) is adapted to control the illuminants (3) such that the ones of the illuminants (3), the respective light beam (I, I, II') of which intersects the central axis (7) at a distance of the surgical field or which is directed to a point, the distance of which is closest to the distance of the surgical field, are operated with a performance increased with respect to the remaining illuminants (3) in order to focus the light beams (I, II, II') on the surgical site.

8. Surgical lamp according to claim 7, wherein
the control device (4) is adapted to control the illuminants (3) such that, alternatively, the ones of the illuminants (3), the light beam (I, II, II') of which intersects the central axis (7) at the distance of the surgical field, as well as the ones, the light beam (I, II, II') of which does not intersect the central axis (7) at the distance of the surgical field, are operated with a performance geared to each other in order to render a light field diameter adjustable.

9. Surgical lamp according to claim 7 or 8, wherein the control device (4) is adapted to control the illuminants (3) such that an illumination in the center of the surgical field (L₁, L₂) remains constant upon a change of the distance of the surgical field.

10. Surgical lamp according to anyone of the preceding claims, wherein the 3D sensor (6) is adapted to detect a surface structure of the surgical field, and the control device (4) is adapted to determine a topography of the surgical field from the data transmitted by the 3D sensor, and to operate the illuminants (3) according to the topography.

11. Method for operating a surgical lamp according to anyone of the claims 1 to 10 comprising the following steps:
- detecting the spatial position of the at least one object by the 3D sensor (6) and transmitting the corresponding data to the control device (4) ; and
- controlling the illuminants (3) according to the spatial position of the at least one object;
- detecting the size of the at least one object between the lamp body (1) and the surgical field by means of the 3D sensor (6) ;
- dimming or switching off the ones of the illuminants (3), the respective light beam of which is directed to the at least one object by the control device (4) ; and
- operating the illuminants, the light beams of which are directed past the at least one object to the surgical field, with increased performance by the control device (4);
and **characterized by**
- predetermining a position of the at least one object at the certain point of time from the spatial position and the motion and/or a contour of the at least one object by means of the control device (4) ; and
- controlling the illuminants (3) depending on the predetermined position and the size of the at least one object so that the ones of the illuminants (3), the respective light beam (I, II, II') of which is directed to the at least one object at the predetermined position, are dimmed or switched off at the certain point of time, and the ones of the illuminants (3), the respective light beam (I, II, II') of which is directed past the at least one object at the predetermined position to the surgical field, are operated with increased performance.

12. Method according to claim 11 with a surgical lamp according to claim 3 or 4, wherein the predetermined position is predetermined from the direction, the velocity, or the acceleration of the at least one object by the control device (4).

13. Method according to anyone of the claims 11 or 12 with a surgical lamp according to claim 5 with the step:
- controlling the illuminants (3) according to a direction, a trajectory, a velocity, or an acceleration of the motion of the at least one object by the control device (4).

14. Method according to anyone of the claims 11 to 13 with a surgical lamp according to anyone of the claims 7 to 10 with the step
- controlling the illuminants (3) such that the ones of the illuminants (3), the light beam (I, II, II') of which intersects the central axis (7) at the distance of the surgical field or is directed to the point, the distance is closest to the distance of the surgical field, are operated with a performance increased with respect to the remaining illuminants (3) in order to focus the light beams (I, II, II') on the surgical site.

15. Method according to anyone of the claims 11 to 14 with a surgical lamp according to anyone of the claims 6 to 9 with the step:
- controlling the illuminants (3) such that the ones of the illuminants (3), the light beam of which intersects the central axis (7) at the distance of the surgical field, as well as the ones of the illuminants (3), the light beam (I, II, II') of which does not intersect the central axis (7) at the distance of the surgical field, are operated with a performance geared to each other by the control device (4) in order to render a light field diameter adjustable.

16. Method according to anyone of the claims 11 to 15 with a surgical lamp according to anyone of the claims 6 to 9 with the step:
- controlling the illuminants (3) such that an illumination in the center of the surgical field remains constant upon a change of the distance of the surgical field.

17. Method according to anyone of the claims 11 to 16 with a surgical lamp according to claim 9 with the steps:
- detecting a topography from the data transmitted by the 3D sensor (6) by the control device (4) ;
- operating the illuminants (3) by the control device (4) such that the surgical field is illuminated according to the topography.

18. Method according to claim 17, wherein the surgical field is illuminated by the illuminants (3) generating a light field (L₁, L₂) with a large diameter when the surgical field is widespread, and the surgical field (L₁, L₂) is illuminated by the illuminants (3) generating a light field with a small diameter when the surgical field is small so that the diameter of the light field (L₁, L₂) basically corresponds to the size of the surgical field.

19. Method according to claim 17 or 18, wherein the surgical field is illuminated by the illuminants (3), the light beams (I, II, II') of which are as vertically as possible directed onto the surgical field when the surgical field includes a deep wound.

20. System of several surgical lamps with at least one surgical lamp according to anyone of the claims 1 to 10, wherein the control devices (4) of several surgical lamps are adapted to be controlled via the data of the 3D sensor (6).

## Revendications

1. Eclairage opératoire pour l'éclairage d'un champ opératoire sur un corps humain avec
un dispositif de commande (4),
un corps d'éclairage (1), qui présente
plusieurs lampes (3) avec chacune un faisceau lumineux (I, II, II') dirigé sur le champ opératoire, et
un capteur 3D (6) pour détecter une position spatiale d'au moins un objet, et
des moyens pour mettre sous tension et hors tension et soumettre à une gradation les lampes (3),
où le dispositif de commande (4) commande les moyens pour mettre sous tension et hors tension et soumettre à une gradation les lampes (3),
le capteur 3D (6) détecte la position spatiale et une taille du au moins un objet entre le corps d'éclairage (1) et le champ opératoire et envoie des données correspondantes au dispositif de commande (4),
le dispositif de commande (4) est adapté pour
commander les lampes (3) d'une manière correspondant à la position spatiale du au moins un objet, et
sur la base de la position spatiale et de la taille du au moins un objet, soumettre à une gradation ou mettre hors tension les lampes (3) dont le faisceau lumineux (I, II, II') est dirigé sur le au moins un objet, et actionner avec une plus grande puissance les lampes (3) dont le faisceau lumineux (I, II, II') est dirigé sur le champ opératoire en passant au voisinage du au moins un objet, où
le capteur 3D (6) est adapté pour détecter un mouvement du au moins un objet, et le dispositif de commande (4) est adapté pour commander les lampes (3) d'une manière correspondant au mouvement du au moins un objet,
et
**caractérisé en ce que**
le dispositif de commande (4) est adapté pour déterminer à l'avance une position prévisible du au moins un objet à un instant déterminé à partir de la position spatiale et du mouvement, et/ou d'un contour du au moins un objet, et pour commander les lampes (3) en fonction de la position déterminée à l'avance et de la taille du au moins un objet, de manière qu'à l'instant déterminé, les lampes (3) dont le faisceau lumineux (I, II, II') est dirigé sur le au moins un objet dans la position déterminée à l'avance soient soumises à une gradation ou soient mises hors tension, et à actionner avec une plus grande puissance les lampes (3) dont le faisceau lumineux (I, II, II') est dirigé sur le champ opératoire en passant au voisinage du au moins un objet dans la position déterminée à l'avance.

2. Eclairage opératoire selon la revendication 1, où le dispositif de commande (4) est adapté pour déterminer, à partir d'un contour du au moins un objet, un domaine du champ opératoire qui doit être éclairé plus fortement, et le dispositif de commande (4) est adapté pour actionner avec une plus grande puissance les lampes (3) dont le faisceau lumineux (I, II, II') est dirigé sur le domaine.

3. Eclairage opératoire selon la revendication 1 ou 2, où le dispositif de commande (4) est adapté pour déterminer à l'avance la position déterminée à l'avance à partir d'une direction du mouvement du au moins un objet.

4. Eclairage opératoire selon l'une des revendications 1 à 3, où le dispositif de commande est adapté pour déterminer à l'avance la position déterminée à l'avance à partir d'une vitesse du mouvement du au moins un objet.

5. Eclairage opératoire selon l'une des revendications 1 à 3, où le dispositif de commande est adapté pour déterminer à l'avance la position déterminée à l'avance à partir d'une accélération du mouvement du au moins un objet.

6. Eclairage opératoire selon l'une des revendications 1 à 5, où le dispositif de commande (4) est adapté pour commander les lampes (3) d'une manière correspondant à une direction, une trajectoire, une vitesse ou une accélération du mouvement du au moins un objet.

7. Eclairage opératoire selon l'une des revendications précédentes,
où l'un des objets est le corps humain sur lequel se trouve le champ opératoire,
la position spatiale correspond à la distance du champ opératoire au corps d'éclairage (1),
le corps d'éclairage (1) présente un axe médian (7),
les faisceaux lumineux (I, II, II') sont dirigés sur des points d'intersection (P_{I}, P_{II}) avec l'axe médian (7) qui ont des distances différentes avec le corps d'éclairage (1),
les faisceaux lumineux (I, II, II') sont superposés pour former un champ lumineux (L₁, L₂) sur le champ opératoire, et
le dispositif de commande (4) est adapté pour commander les lampes (3) de telle manière que les lampes (3) dont le faisceau lumineux (I, II, II') coupe l'axe médian (7) à distance du champ opératoire, ou qui est dirigé sur un point dont la distance est la plus proche de la distance du champ opératoire, sont actionnées avec une plus grande puissance que les autres lampes (3), pour focaliser les faisceaux lumineux (I, II, II') sur le site opératoire.

8. Eclairage opératoire selon la revendication 7, où
le dispositif de commande (4) est adapté pour commander les lampes (3) de telle manière que les lampes (3) dont le faisceau lumineux (I, II, II') coupe l'axe médian (7) à distance du champ opératoire ainsi que celles dont le faisceau lumineux (I, II, II') ne coupe pas l'axe médian (7) à distance du champ opératoire sont actionnées alternativement avec une puissance ajustée l'une à l'autre pour rendre réglable un diamètre du champ lumineux.

9. Eclairage opératoire selon la revendication 7 ou 8, où le dispositif de commande (4) est adapté pour commander les lampes (3) de telle manière qu'une intensité d'éclairage reste constante au centre du champ opératoire (L₁, L₂) lors d'une modification de la distance du champ opératoire.

10. Eclairage opératoire selon l'une des revendications précédentes, où le capteur 3D (6) est adapté pour détecter une structure superficielle du champ opératoire et le dispositif de commande (4) est adapté pour déterminer une topographie du champ opératoire à partir des données envoyées par le capteur 3D et pour actionner les lampes (3) d'une manière correspondant à la topographie.

11. Procédé pour actionner un éclairage opératoire selon l'une des revendications 1 à 10, qui présente les étapes suivantes
- détection de la position spatiale du au moins un objet par le capteur 3D (6) et envoi des données correspondantes au dispositif de commande (4) ;
- commande des lampes (3) d'une manière correspondant à la position spatiale du au moins un objet ;
- détection de la taille du au moins un objet entre le corps d'éclairage (1) et le champ opératoire au moyen du capteur 3D (6) ;
- gradation ou mise hors tension des lampes (3) dont le faisceau lumineux est dirigé sur le au moins un objet par le dispositif de commande (4) ; et
- actionnement avec une plus grande puissance des lampes dont les faisceaux lumineux sont dirigés sur le champ opératoire en passant au voisinage du au moins un objet par le dispositif de commande (4) ; et **caractérisé par**
- la détermination à l'avance d'une position du au moins un objet à l'instant déterminé à partir de la position spatiale et du mouvement et/ou d'un contour du au moins un objet au moyen du dispositif de commande (4) ; et
- la commande des lampes (3) en fonction de la position déterminée à l'avance et de la taille du au moins un objet, de manière qu'à l'instant déterminé, les lampes (3) dont le faisceau lumineux (I, II, II') est dirigé sur le au moins un objet dans la position déterminée à l'avance soient soumises à une gradation ou soient mises hors tension, et à actionner avec une plus grande puissance les lampes (3) dont le faisceau lumineux (I, II, II') est dirigé sur le champ opératoire en passant au voisinage du au moins un objet dans la position déterminée à l'avance.

12. Procédé selon la revendication 11 avec un éclairage opératoire selon la revendication 3 ou 4, où la position déterminée à l'avance à partir de la direction, de la vitesse ou de l'accélération du au moins un objet est déterminée à l'avance par le dispositif de commande (4).

13. Procédé selon l'une des revendications 11 ou 12, avec un éclairage opératoire selon la revendication 5 avec l'étape
- commande des lampes (3) d'une manière correspondant à une direction, une trajectoire, une vitesse ou une accélération du mouvement du au moins un objet par le dispositif de commande (4).

14. Procédé selon l'une des revendications 11 à 13 avec un éclairage opératoire selon l'une des revendications 7 à 10, avec l'étape
- commande des lampes (3) de telle manière que les lampes (3) dont le faisceau lumineux (I, II, II') coupe l'axe médian (7) à distance du champ opératoire, ou est dirigé sur le point dont la distance est la plus proche de la distance du champ opératoire, sont actionnées avec une plus grande puissance que les autres lampes (3), pour focaliser les faisceaux lumineux (I, II, II') sur le site opératoire.

15. Procédé selon l'une des revendications 11 à 14 avec un éclairage opératoire selon l'une des revendications 6 à 9, avec l'étape
- commande des lampes (3) de telle manière que les lampes (3) dont le faisceau lumineux coupe l'axe médian (7) à distance du champ opératoire ainsi que les lampes (3) dont le faisceau lumineux (I, II, II') ne coupe pas l'axe médian (7) à distance du champ opératoire sont actionnées par le dispositif de commande (4) avec une puissance ajustée l'une à l'autre pour rendre réglable un diamètre du champ lumineux.

16. Procédé selon l'une des revendications 11 à 15 avec un éclairage opératoire selon l'une des revendications 6 à 9, avec l'étape
- commande des lampes (3) de telle manière qu'une intensité d'éclairage reste constante au centre du champ opératoire lors d'une modification de la distance du champ opératoire.

17. Procédé selon l'une des revendications 11 à 16 avec un éclairage opératoire selon la revendication 9, avec les étapes
- détection d'une topographie par le dispositif de commande (4) à partir des données envoyées par le capteur 3D (6) ;
- actionnement des lampes (3) par le dispositif de commande (4) de telle manière que le champ opératoire est éclairé d'une manière correspondant à la topographie.

18. Procédé selon la revendication 17, où le champ opératoire est éclairé par les lampes (3) qui forment un champ lumineux (L₁, L₂) avec un grand diamètre quand le champ opératoire est de grande surface, et le champ opératoire est éclairé par les lampes (3) qui forment un champ lumineux (L₁, L₂) avec un petit diamètre quand le champ opératoire est petit, de sorte que le diamètre du champ lumineux (L₁, L₂) correspond sensiblement à la taille du champ opératoire.

19. Procédé selon la revendication 17 ou 18, où le champ opératoire est éclairé par des lampes (3) dont les faisceaux lumineux (I, II, II') sont dirigés sur le champ opératoire le plus perpendiculairement possible, quand le champ opératoire contient une plaie profonde.

20. Système à plusieurs éclairages opératoires avec au moins un éclairage opératoire selon l'une des revendications 1 à 10, où les dispositifs de commande (4) de plusieurs éclairages opératoires sont adaptés pour être commandés par le biais des données du capteur 3D (6).
